# EUROPEAN PATENT SPECIFICATION

(11) **EP 2 996 739 B1**
(45) Date of publication and mention of the grant of the patent: **30.05.2018**
(21) Application number: 14724128.5
(22) Date of filing: 10.04.2014
(51) Int. Cl.: A61M 5/00, A61M 5/32, B65B 55/08, B65B 5/02, B65B 7/28

(54) **NEEDLE TIP STORAGE AND REMOVAL DEVICE AND METHODS OF MANUFACTURE THEREOF**
AUFBEWAHRUNGS- UND ENTNAHMEVORRICHTUNG FÜR NADELSPITZEN UND VERFAHREN ZUR HERSTELLUNG DAVON
DISPOSITIF D'ENTREPOSAGE ET D'ÉLIMINATION DE POINTE D'AIGUILLE ET PROCÉDÉS DE FABRICATION CORRESPONDANTS

(30) Priority: 11.04.2013 GB 201306601
(43) Date of publication of application: 23.03.2016
(60) Divisional application: 18158443.4
(73) Proprietor: Owen Mumford Limited, Oxford, Oxfordshire OX20 1TU (GB)
(72) Inventor: EVANS, Timothy, Oxford Oxfordshire OX20 1TU (GB); HAWKINS, Alan, Oxford Oxfordshire OX20 1TU (GB); COWE, Toby, Oxford Oxfordshire OX20 1TU (GB); VARDE, Andrew John, Oxford, Oxfordshire OX20 1TU (GB)
(74) Representative: Wynne-Jones, Lainé and James LLP
(86) International application number: PCT/GB2014/051125
(87) International publication number: WO 2014/167340

(56) References cited:
- EP-A1- 2 298 397
- EP-A1- 2 522 380
- GB-A- 2 437 923
- US-A- 5 554 129

## Description

This invention relates to needle tip storage and removal devices and to methods of manufacture thereof. Many drugs that need to be injected regularly are delivered by a pen injector or the like (such as our Autopen® injector) which has a removable and disposable needle tip (or "pen tip") which is screwed and unscrewed or otherwise attached and detached by rotary action to the front end of the pen injector to provide a fresh needle for each injection. An example of a typical needle tip is our UniFine® needle tip. This comprises an internally threaded cylindrical hub typically of a plastics material and having a fluted or splined outer cylindrical surface. A needle projects axially forwardly of the hub.

It is known to supply such needle tips in sterile storage containers sealed by means of a foil seal or the like. The storage container has internal splines which engage the external splines on the outer cylindrical surface of the needle tip. The storage container can therefore be used as a spanner or wrench to screw the needle tip into engagement with a threaded portion on the pen injector where this is provided and which can be slid on or off the needle tip. The storage container may provide a similar function in other types of rotary connection. It is preferred that the user, having applied a needle tip to a pen injector and withdrawn the needle tip container to expose the needle tip, should keep the container safe so that it can be used to re-cover the needle. This is so that the container can be used again as a spanner or wrench to unscrew it from the pen injector and then to allow safe disposal of the used needle tip inside the storage container, and also so that the needle is safely covered as the needle tip is being unscrewed.

However, this practice is not always followed with the result that the user may try and remove an unshrouded, used needle tip by hand, with the attendant risk of needle stick injury.

GB2437923 is designed to address this problem and provides a needle tip storage and removal device having opposed, oppositely directed storage and removal compartments, intended to be moulded in one piece in an injection moulding process.

We have found however, this arrangement may be further improved. In particular, in a typical manufacturing facility for such devices, the facility will often be required to switch between insertion, sealing and sterilising a needle tip in a conventional, single container, as well as to be modified to produce runs of needle tips each sealed and sterilised in a storage compartment of a double container of the types shown in GB2437923. This however, requires considerable rejigging of the machinery and can result in a day or so of down time, with the consequent loss in production. In addition, because such devices are normally sterilised by exposure to gamma radiation, and the extent and/or duration of the exposure required is dependant on the mass and volume of material in the device, some of the dosage of radiation is 'wasted' on the integrally formed removal compartment, which does not actually need to be sterilised. Also, because the removal tool needs to be designed to have robust puncture resistance, the integrally formed storage and removal compartments are typically made of a material which is puncture resistant. The puncture resistance material tends to be thicker and so increases the amount of material used which is undesirable for a disposable item.

Other publications include EP2298397A1, US5554129A, and EP2522380A1.

Accordingly, we have designed a needle tip storage and removal device which allows the materials for the storage compartment to be optimised having regard to the requirements of that compartment, namely maintaining sterility and ease of sterilisation by radiation and the materials of the removal tool to be optimised for its purpose, typically good puncture resistance and good resilience, economy of production and disposability.

According to the present invention there is provided a needle tip storage and removal device for use with a needle tip as defined in the appended independent claim. Further preferable features are defined in the appended dependent claims.

Whilst the invention has been described above, it extends to any inventive combination or sub-combination of the features set out above, and in the following description, claims or appended drawings.

The invention may be performed in various ways and, by way of example only, various embodiments thereof will now be described in detail, reference being made to the accompanying drawings in which:
Figures 1 (a) and (b) are perspective views of a needle tip storage and removal device, with the main components before and after assembly respectively;
Figures 2(a) and (b) are section views of a second embodiment of needle tip storage and removal device before and after assembly respectively;
Figures 3(a) and (b) are section views of a third embodiment of needle tip storage and removal device before and after assembly respectively;
Figures 4(a) and (b) are section views of a fourth embodiment of needle tip storage and removal device before and assembly respectively;
Figures 5(a) to (d) are views of a fifth embodiment of needle tip storage and removal device;
Figures 6(a) to (c) are views of a sixth embodiment of needle tip storage and removal device;
Figures 7(a) to (c) are views of a seventh embodiment of needle tip storage and removal device;
Figures 8(a) to (c) are views of an eighth embodiment of needle tip storage and removal device, prior to insertion of the needle tip storage container;
Figures 9(a) and (b) are views of a ninth embodiment of needle tip storage and removal device in the as supplied and locked conditions respectively;
Figures 10(a) and (b) are exploded section and perspective views respectively of the ninth embodiment of needle tip storage and removal device, and
Figures 11 (a) to (j) are successive views showing use of the ninth embodiment of needle tip storage and removal device.

The various embodiments described herein provide needle tip storage and removal devices made up of a storage container or compartment containing a needle tip and hermetically sealed by a removable foil or the like, the needle tip container then being assembled with a removal housing which is or has been formed in a separate forming step. This means that the needle tip storage and removal device may be made by taking a pre-existing needle tip container sealed with the needle tip inside it, and then assembling it with the removal housing. In this way, the moulding of the storage container, loading of the needle tip and subsequent sealing of the container may be optimised for subsequent sterilisation process by gamma irradiation prior to assembly with the removal housing. The sterile needle tip storage container, with needle tip inside, may then be assembled in a sterile manner with the removal housing.

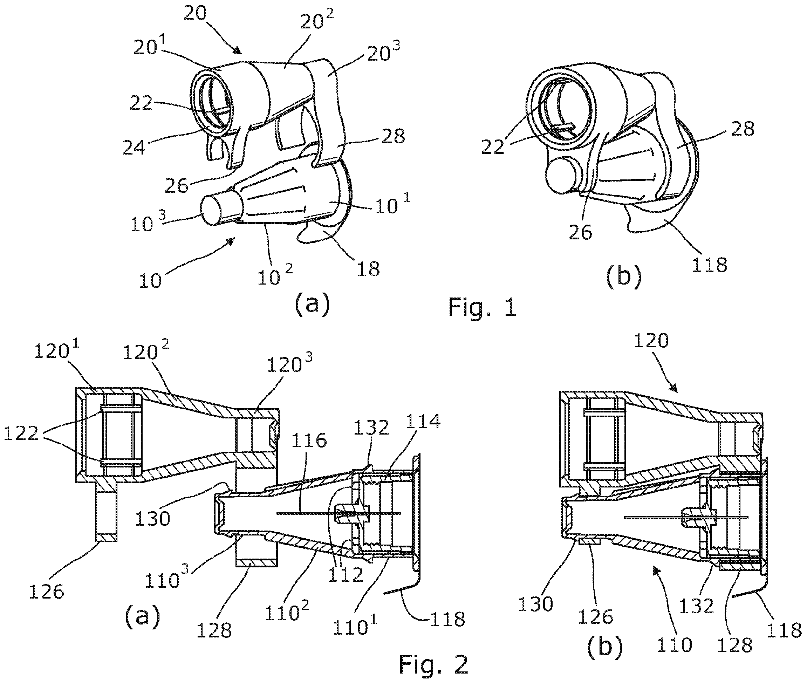

In the embodiments below, the needle tip storage container is made up of a relatively large diameter portion 10', which merges with a frustoconical portion 10² which itself merges with a closed tip portion 10³ of relative small diameter, as seen for example in Figures 1(a), 5(a) and 10(b). Internally, the larger cylindrical portion defines an internal cylindrical wall with splines 22 equi-spaced around it, the splines being designed to allow the needle tip hub to be slid into and out of non-rotatable engagement with the needle tip storage container. In this example, the needle tip has a double ended needle (see Figure 2(a)). The needle tip storage container 10 is sealed by a removable foil 18. In the first embodiment, the needle tip removal housing 20 is formed in a separate process and from, typically, different materials but has a similar overall shape and internal shape as that of the needle tip storage container 10. Thus the needle tip removal housing 20 has a relatively large diameter portion 20¹ that merges with a frustoconical portion 20² which itself merges with a cylindrical tip portion 20³. Internally, the large cylindrical portion 20¹ is provided with splines 22 into which a used needle tip may be slid in non-rotatable engagement. The open end of the larger diameter cylindrical portion 20¹ is provided with an internal snap rib 24 designed to lock a used needle tip in the removal housing 20 when it is fully pushed home.

Projecting transversely from the large cylindrical portion 20¹ and the small cylindrical portion 20³ are respective small and large clip portions 26, 28 respectively defining resilient clip recesses which allow the needle tip storage container and the removal housing to be clipped together as shown in Figure 1(b). The clipping action may be permanent or reversible.

Referring now to Figure 2, many features of the second embodiment are similar to those of the first embodiment and will be given similar reference numbers but incremented by 100. In this embodiment, the needle tip removal housing is provided with laterally extending small and large ring portions 126 and 128 respectively. The needle tip storage container is provided with small and large diameter snap rings 130, 132 on the external surface of the small and large diameter cylindrical portions 110³ and 110¹. The needle tip storage container and the needle tip removal housing may be assembled together by disposing them in the position of Figure 2(a) and then bringing them together so that the ribs 130, 132 squeeze through and snap through the ring shaped portions 126 and 128, as seen in Figure 2(b).

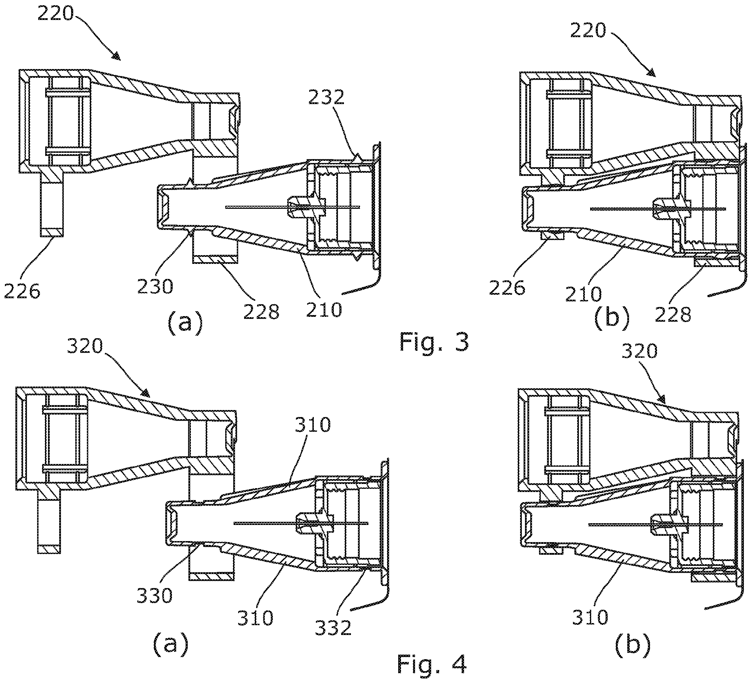

Referring now to Figure 3, many features of the third embodiment are similar to those of the second embodiment and will be given similar reference numbers but incremented by 100. In this embodiment, the small and large diameter portions of the needle tip storage container 210 are formed with annular ribs 230 and 232. The needle tip storage container 210 and the needle tip removal housing 220 are positioned as shown in Figure 3 and then pushed together so that the annular ribs 230 and 232 contact and pass into the inner cylindrical surface of rings 226, 228 on the needle tip removal housing 220. An ultrasonic welding process is then applied to cause the ribs 230 and 232 to fuse or melt with surrounding ring portion 226, 228 permanently to secure the needle tip storage container and the needle tip removal housing together.

Referring now to Figure 4, many features of the fourth embodiment are similar to those of the third embodiment and will be given similar reference numbers but incremented by 100. In this embodiment, the needle tip storage container 310 is provided with annular recesses 330 and 332 in its small and large diameter portions respectively. The needle tip removal housing 320 is identical to that of the fourth embodiment and the needle tip storage container and the needle tip removal housing are assembled together by applying an adhesive to the grooves 330 and 332 and then assembling the two together as shown in Figure 4(b).

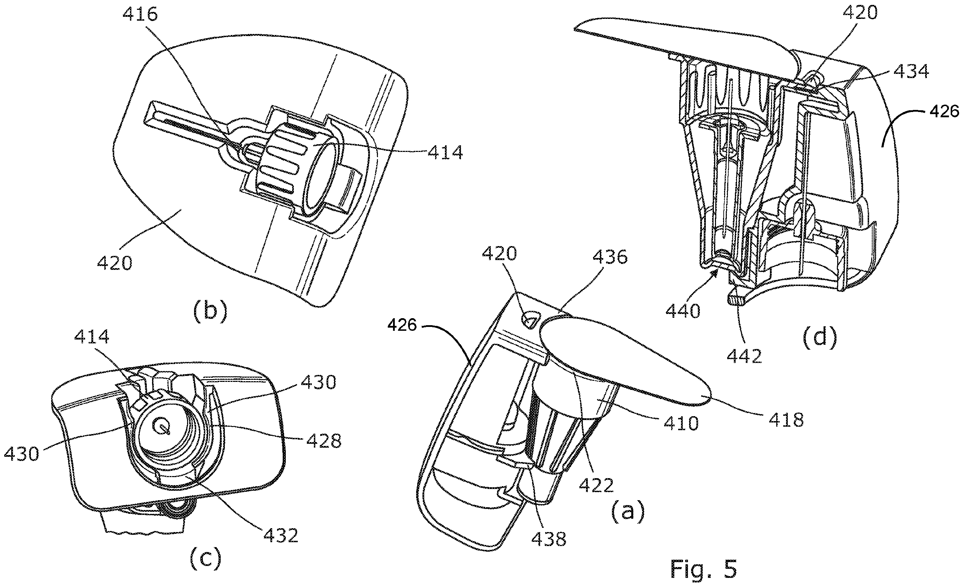

Referring now to Figure 5, in this embodiment a needle tip storage container 410 similar to that of the previous embodiment, containing a needle tip of the type described above and sealed by a foil 418 is provided with a tab 420 that extends transversely from a flange 422 provided at the open end of the larger cylindrical portion of the needle tip storage container. The flange 422 provides a planar radial surface to which the foil 418 is heat-sealed. The tab 420 is formed integrally in the same moulding process as the main portion of the needle tip storage container 410.

In this embodiment, the needle tip removal housing is based on our existing UniGuard® device, as described in WO 2005/102424.

The needle tip removal housing 426 has an opening 428 of outline generally matching and adapted to receive the hub 414 and needle 416 of a needle tip when inserted laterally. The opening includes a U-shaped cradle portion 428 which has two abutments 430 past which the needle hub snaps when inserted laterally. Once in its fully home position, the needle hub engages splines (not shown) on the interior of the U-shaped portion 428 to engage it non-rotatably. If required, there may be a two stage engagement action whereby the user initially inserts the needle hub laterally and then pushes it axially (in the direction of the needle) to a fully home position. In this latter position, the needle hub may snap past a tongue 432 to hold it against reverse axial movement. As seen particularly in Figures 5(a) and5(d), the needle removal housing 426 is provided with a recess 434 into which the tab 420 on the flange of the needle tip storage container be snapped irreversibly. Snapping the tab 420 into the recess 434 holds the needle tip storage container closely against the underside of the needle tip removal housing 426, with portions thereof cradled by supports 436, 438 and the smaller diameter end of the needle housing is provided with a dished portion 440 over into the edge of which hooks a protrusion 442 to hold the needle tip storage container and the needle tip removal housing securely together.

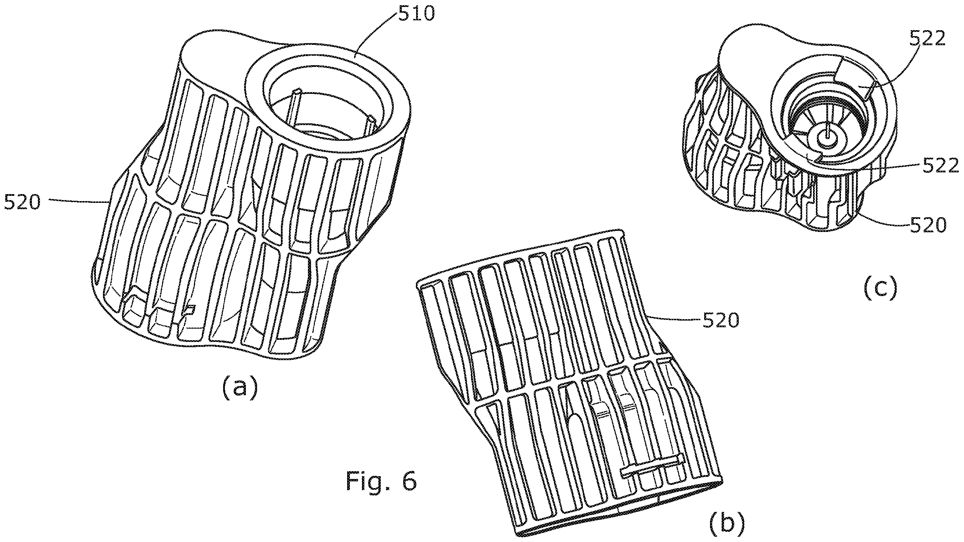

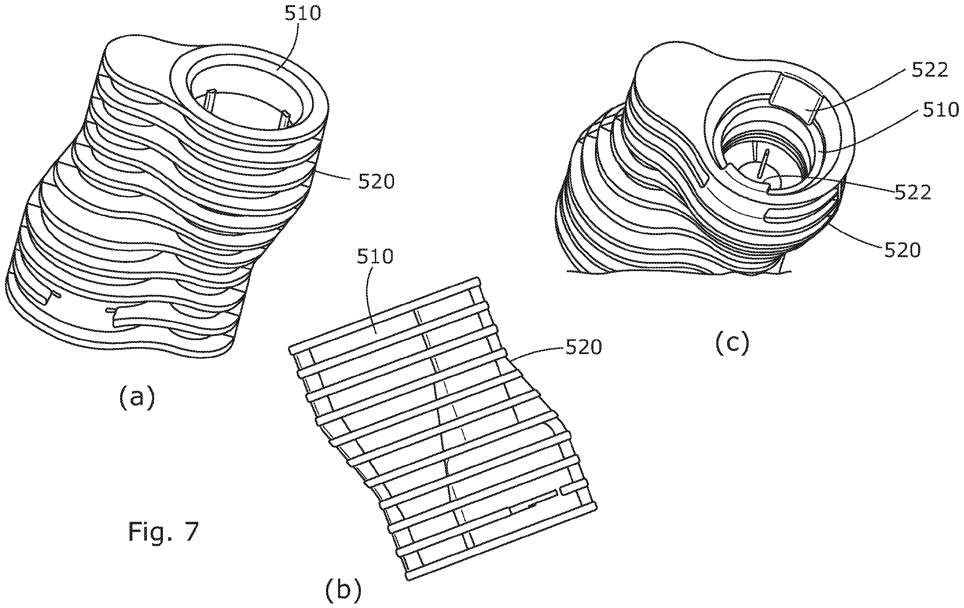

Referring now to the embodiments of Figures 6 and 7, here a two-shot or overmoulding process is used to initially form a needle tip storage container 510 of similar form to those of the earlier embodiments which can then be fitted with a needle tip sealed and then sterilised. In the second moulding step, using a different plastics material, a needle tip removal housing 520 is moulded which has a main needle tip receiving portion with an integral frame structure embracing and connecting the needle tip storage container. The needle tip removal portion may include flexible tabs 522 to prevent removal of a used needle tip once inserted into the needle tip removal portion. As with the other embodiments, the needle tip removal portion has a recess configured non-rotatably to receive a used needle tip. In Figure 6, the main frame elements encasing the needle tip storage container run generally longitudinally, whereas in the arrangement of Figure 7, they run generally circumferentially.

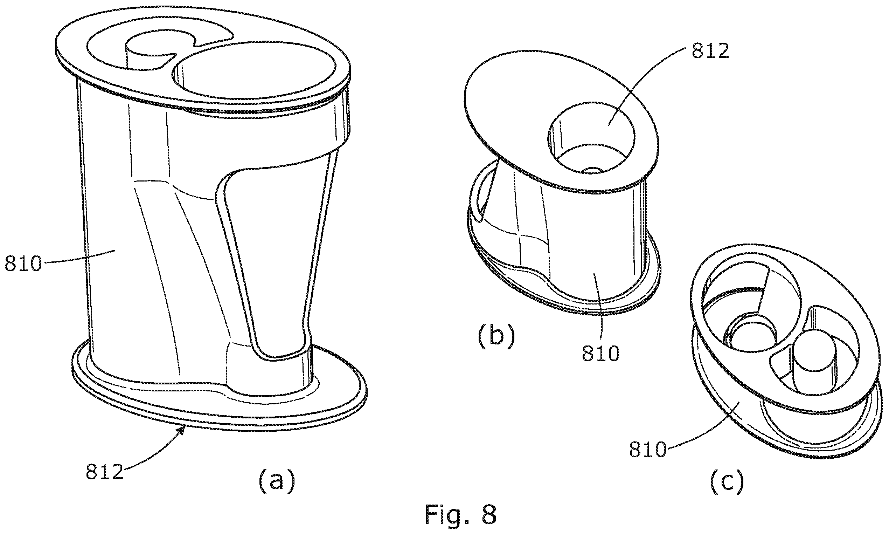

Referring now to Figure 8, in this embodiment a needle tip removal housing 810 is provided with a needle tip removal portion 812 designed non-rotatably to receive a used needle tip as before. Adjacent to the needle tip removal portion there is a shaped recess 814 designed to receive by a snap fit a needle tip storage container. As previously, therefore, each of the needle tip removal housing and a needle tip storage container may be separately formed and then assembled together to form the final product.

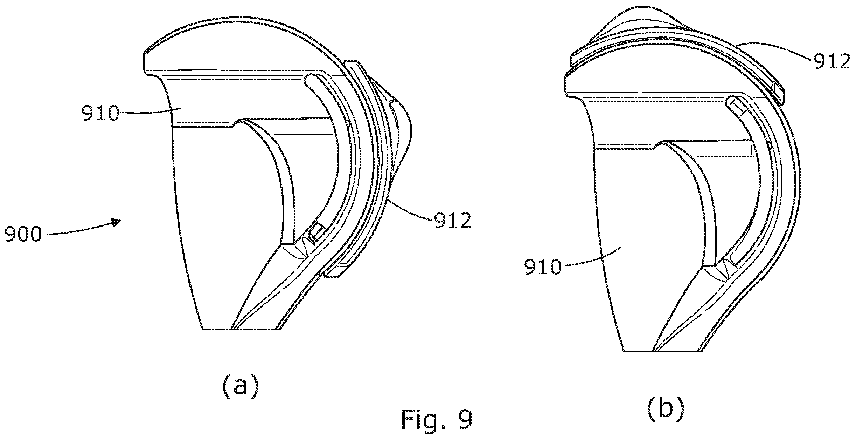

Referring now to the embodiment illustrated in Figures 9 to 11, here the needle tip storage and removal device is made up by assembling a needle tip within the needle tip storage container to provide sterile primary packaging as previously, with that assembly then being fitted into a disposal system 900, thereby allowing broader material selection for the disposal system and reducing processing costs. The needle disposal system comprises a housing 910 with a moveable 912 lid that can be moved from an open position, in which access to the needle tip storage container is allowed, and a closed position in which access to the needle storage container is obstructed. The device may use any suitable form of closing movement but, in the embodiment of Figures 9 to 11, an arcuate sliding movement is used to slide a cover from the open position shown in Figure 9(a) to the closed position shown in Figure 9(b). The cover includes a latching mechanism 914, 916 to latch it in its closed position.

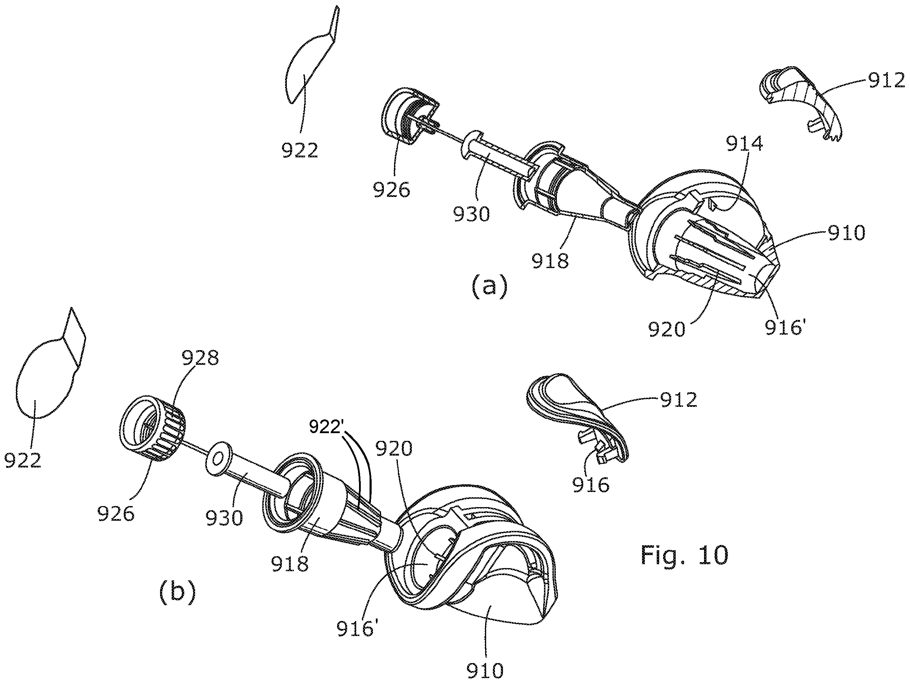

Referring now to Figure 10, the housing 910 has a recess 916' designed to receive the needle tip storage container 918. The needle tip storage container may be an interference fit within the recess or it may snap into place. As seen more particularly in Figure 10(a), the recess is provided with stepped internal radial ribs 920 which co-operate with formations 922' on the outside of the needle tip storage container to prevent rotation there between. The latching mechanism for locking the cover 912 in its closed position comprises a tooth 914 on the housing which co-operates with the corresponding flexible tooth on the cover 916. The device of Figure 10 is designed so that, when a needle storage container 918 is fitted into the recess, the foil 922 on the needle storage container 918 obstructs movement of the cover 912 towards its closed position, so that the cover can only be slid closed once the foil has been removed.

Referring again to Figure 10(a), the radial ribs 920 are stepped inwardly and designed so that, in the event that the needle tip storage container should somehow become dislodged from the disposal system, as a failsafe arrangement the recess can slideably receive a non-rotatable fashion the needle hub 926, with the inwardly stepped ribs 920-co-operating with the splines 928 on the hub.

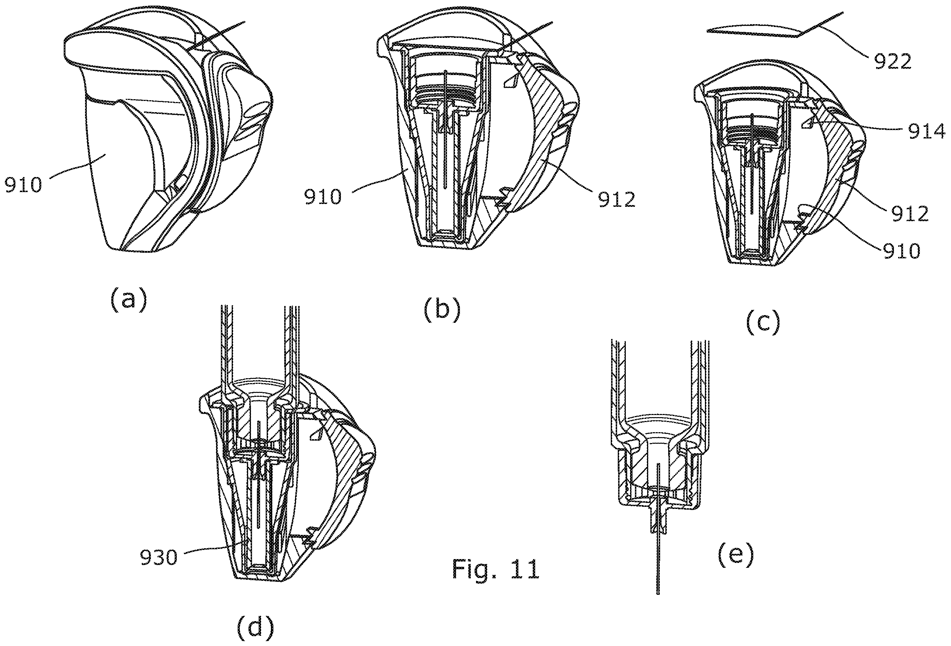

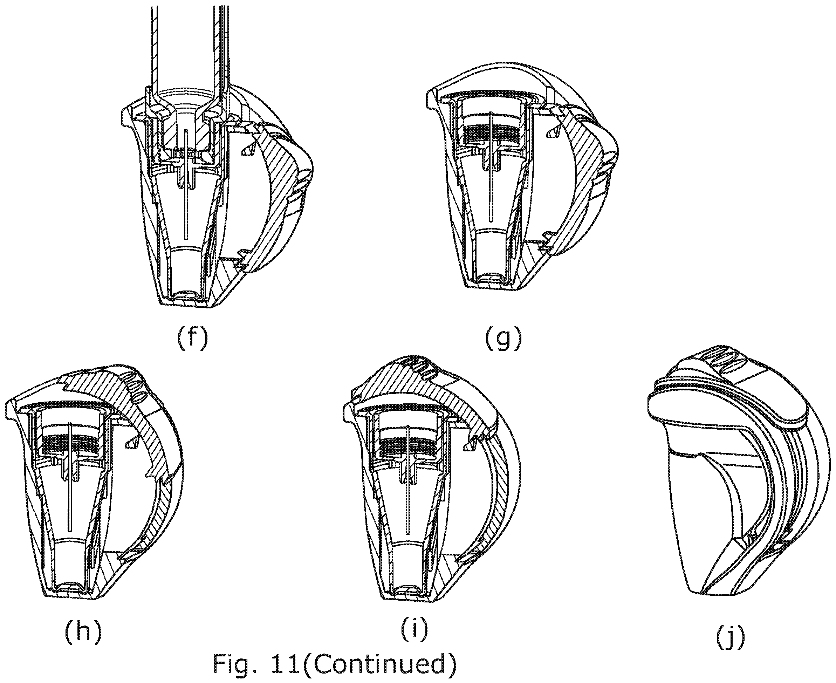

Referring to Figure 11, when the device is as supplied, the cover 912 is in its open position and prevented from moving to the closed position by the tab of the foil seal 922. The user prepares the device by removing the foil seal 922 and then inserting a pen and screwing it on to the needle to collect the needle. The user then withdraws the pen from the device and then removes an inner needle shield 930 to expose the needle ready for an injection (Figure 11 (e)). After the injection, the user replaces the needle hub into the needle storage container and unscrews the pen. This can be seen in Figure 11 (f). As an additional feature, should the primary packaging have fallen out, the pen can be inserted deeper into the disposal system to engage the needle hub with the radial splines. Having removed the pen, the cover may be slid around the body to cover the needle with the tooth on the cover snapping past the tooth on the housing to lock it in place. The used needle is then in a safe condition ready for disposal.

## Claims

1. A needle tip storage and removal device for use with a needle tip comprising a cylindrical hub (926) and a needle extending axially therefrom, which comprises a storage compartment (918) formed in a given forming process for containing a needle tip in a sterile environment and for being sealed by an openable seal element (922), a separately formed outer housing (910) having a recess (916') adapted to receive said storage compartment (918), and a closure element (912) configured to be moveable between a retracted position in which access to the interior of said storage compartment (918) is allowed, and a closed position in which such access is prevented or inhibited, wherein the recess (916') is provided with stepped internal radial ribs (920) which co-operate with formations (922') on the outside of the needle tip storage container to prevent rotation there between.

2. A needle tip storage and removal device according to Claim 1, wherein said storage compartment (918) defines an interior recess (916') into which a needle tip may be inserted for non-rotatable engagement, and said outer housing (910) defines a stepped recess having a larger diameter cylindrical portion for receiving said storage compartment (918) and, beyond said larger diameter cylindrical portion, a smaller diameter cylindrical portion into which a needle tip may be inserted for non-rotatable engagement if the larger diameter cylindrical recess is not occupied by a storage compartment.

3. A needle tip storage and removal device according to Claim 1, wherein said storage compartment (918) includes a removable seal element (922), and said outer housing (910) and/or said closure element (912) are configured so that the said seal element (922), if present on said storage compartment (918), blocks movement of said closure element (912) to its closed position, but when said seal element (922) is absent said closure element (912) may be moved to its closed position.

## Patentansprüche

1. Vorrichtung zum Aufbewahren und Entfernen einer Nadelspitze bei Verwendung mit einer Nadelspitze, umfassend eine zylindrische Nabe (926) und eine Nadel, die sich in axialer Richtung von der Nabe erstreckt, ferner mit einer Aufbewahrungskammer (918), hergestellt in einem gegebenen Formungsprozeß, um eine Nadelspitze in einer sterilen Umgebung aufzunehmen und um durch ein zu öffnendes Dichtungselement (922) abgedichtet zu werden, ferner umfassend ein getrennt geformtes äußeres Gehäuse (910) mit einer Aussparung (916'), die in der Lage ist, die Aufbewahrungskammer (918) aufzunehmen, und mit einem Verschlußelement (912), das so gebaut ist, daß es zwischen einer zurückgezogenen Lage, in der das Innere der Aufbewahrungskammer (918) zugänglich ist, und einer geschlossenen Lage, in der ein solcher Zugang verhindert oder gehemmt wird, beweglich ist, wobei die Aussparung (916') mit gestuften inneren, radialen Rippen (920) versehen ist, die mit Ausbildungen (922') auf der Außenseite der Nadelspitzen-Aufbewahrungskammer zusammenwirken, um zwischen ihnen eine Drehbewegung zu verhindern.

2. Vorrichtung zum Aufbewahren und Entfernen einer Nadelspitze nach Anspruch 1, **dadurch gekennzeichnet, daß** die Aufbewahrungskammer (918) eine innere Aussparung (916') bildet, in die eine Nadelspitze eingesteckt werden kann, so daß sie in einem nicht drehenden Eingriff steht, und daß das äußere Gehäuse (910) eine gestufte Aussparung hat, die einen zylindrischen Abschnitt größeren Durchmessers aufweist, der zur Aufnahme der Aufbewahrungskammer (918) dient, und jenseits des zylindrischen Abschnitts größeren Durchmessers einen zylindrischen Abschnitt kleineren Durchmessers aufweist, in den eine Nadelspitze eingesteckt werden kann, so daß sie in einem nicht drehbaren Eingriff steht, wenn die zylindrische Aussparung größeren Durchmessers nicht durch eine Aufbewahrungskammer ersetzt ist.

3. Vorrichtung zum Aufbewahren und Entfernen einer Nadelspitze nach Anspruch 1, **dadurch gekennzeichnet, daß** die Aufbewahrungskammer (918) ein entfernbares Dichtungselement (922) aufweist, und daß das äußere Gehäuse (910) und / oder das Verschlußelement (912) so gestaltet sind, daß das genannte Dichtungselement (922), wenn es auf der Aufbewahrungskammer (918) vorhanden ist, die Bewegung des Verschlußelementes (912) in seine geschlossene Lage blockiert, jedoch wenn das Dichtungselement (922) nicht vorhanden ist, die Bewegung des Verschlußelementes (912) in seine verschlossene Lage zuläßt.

## Revendications

1. Dispositif d'entreposage et d'élimination de pointe d'aiguille destiné à être utilisé avec une pointe d'aiguille comprenant un raccord cylindrique (926) et une aiguille s'étendant axialement à partir de celui-ci, qui comprend un compartiment d'entreposage (918) formé dans un processus de formage donné pour contenir une pointe d'aiguille dans un environnement stérile et être scellé par un élément de scellement ouvrable (922), un logement externe formé séparément (910) ayant un évidement (916') apte à recevoir ledit compartiment d'entreposage (918), et un élément de fermeture (912) configuré pour être mobile entre une position rétractée dans laquelle un accès à l'intérieur dudit compartiment d'entreposage (918) est autorisé, et une position fermée dans laquelle un tel accès est empêché ou interdit, l'évidement (916') comportant des nervures radiales internes étagées (920) qui coopèrent avec des formations (922') sur l'extérieur du contenant d'entreposage de pointe d'aiguille pour empêcher une rotation entre ceux-ci.

2. Dispositif d'entreposage et d'élimination de pointe d'aiguille selon la revendication 1, dans lequel ledit compartiment d'entreposage (918) définit un évidement intérieur (916') dans lequel une pointe d'aiguille peut être insérée pour un engagement non-rotatif, et ledit logement externe (910) définit un évidement étagé ayant une partie cylindrique de plus grand diamètre pour recevoir ledit compartiment d'entreposage (918) et, au-delà de ladite partie cylindrique de plus grand diamètre, une partie cylindrique de plus petit diamètre dans laquelle une pointe d'aiguille peut être insérée pour un engagement non-rotatif si l'évidement cylindrique de plus grand diamètre n'est pas occupé par un compartiment d'entreposage.

3. Dispositif d'entreposage et d'élimination de pointe d'aiguille selon la revendication 1, dans lequel ledit compartiment d'entreposage (918) comprend un élément de scellement amovible (922), et ledit logement externe (910) et/ou ledit élément de fermeture (912) sont configurés de telle sorte que ledit élément de scellement (922), s'il est présent sur ledit compartiment d'entreposage (918), bloque un mouvement dudit élément de fermeture (912) vers sa position fermée mais, lorsque ledit élément de scellement (922) est absent, ledit élément de fermeture (912) peut être déplacé à sa position fermée.
